# EUROPEAN PATENT SPECIFICATION

(11) **EP 0 477 290 B1**
(45) Date of publication and mention of the grant of the patent: **29.01.1997**
(21) Application number: 90910240.2
(22) Date of filing: 14.06.1990
(51) Int. Cl.: C12P 21/06, C12N 15/00, C12N 5/00, C12N 11/02, C12N 5/02, C12M 3/00, C12M 3/02, C12M 3/04, C12N 1/00

(54) **METHODS, COMPOSITIONS AND DEVICES FOR GROWING CELLS**
VERFAHREN, ZUBEREITUNGEN UND ANORDNUNGEN FÜR ZELLZUCHT
PROCEDES, COMPOSITIONS ET DISPOSITIFS POUR LA CROISSANCE DE CELLULES

(30) Priority: 15.06.1989 US 366639
(43) Date of publication of application: 01.04.1992
(60) Divisional application: 96201874.3
(73) Proprietor: THE REGENTS OF THE UNIVERSITY OF MICHIGAN, Ann Arbor, Michigan 48109-1280 (US)
(72) Inventor: EMERSON, Stephen, G., Ann Arbor, MI 48104 (US); CLARKE, Michael, F., Ann Arbor, MI 48104 (US); PALSSON, Bernhard, O., Ann Arbor, MI 48104 (US)
(74) Representative: Des Termes, Monique
(86) International application number: US9003438
(87) International publication number: WO9015877

(56) References cited:
- EP-A- 0 358 506
- US-A- 4 135 975
- US-A- 4 514 499
- US-A- 4 810 643
- US-A- 4 847 201
- US-A- 4 861 714
- US-A- 4 889 812
- CELL, vol. 47, 10 October 1986, Cambridge, MA (US); YU-CHUNG YANG et al., pp. 3-10
- PROCESS. BIOCHEMISTRY, August 1986; A. MIZRAHI, pp. 108-112
- CANADIAN JOURNAL OF CHEMICAL ENGINEERING, vol. 64, August 1986; S.R. ADAMSON et al., pp. 531-539
- T.M. DESTER et al., "Long Term Bone Marrow Culture", 1984, Alan R. Liss Inc. (publ.); pp. 57-96
- Long-Term Bone Marrow Culture 1984, Alan R. Liss Inc., p. 119 - 131
- J. Tiss. Cult. Meth. 13, 1991, 55 - 62

## Description

### INTRODUCTION

### Technical Field

The field of the invention is the growth of normal mammalian cells in culture.

### Background

There is significant interest in the ability to use cells for a wide variety of therapeutic purposes. The hematopoietic system exemplifies the extraordinary range of cells involved in protection of mammalian hosts from pathogens, toxins, neoplastic cells, and other diseases. The hematopoietic system is believed to evolve from a single stem cell, from which all the lineages of the hematopoietic system derive. The particular manner in which the stem cell proliferates and differentiates to become determined in its lineage is not completely understood, nor are the factors defined. However, once the stem cell has become dedicated to a particular lineage, there appear to be a number of factors, for example colony stimulating factors, which allow, and may direct the stem cell to a particular mature cell lineage.

There are many uses for blood cells. Platelets find use in protection against hemorrhaging, as well as a source of platelet derived growth factor. Red blood cells can find use in transfusions to support the transport of oxygen. Specific lymphocytes may find application in the treatment of various diseases, where the lymphocyte is specifically sensitized to an epitope of an antigen. These and many other purposes may be contemplated.

In order to provide these cells, it will be necessary to provide a means, whereby cells can be grown in culture and result in the desired mature cell, either prior to or after administration to a mammalian host. The hematopoietic cells are known to grow and mature to varying degrees in bone, as part of the bone marrow. It therefore becomes of interest to recreate a system which provides substantially the same environment as is encountered in the bone marrow, as well as being able to direct these cells which are grown in culture to a specific lineage.

### Relevant Literature

U.S. Patent No. 4,721,096 describes a 3-dimensional system involving stromal cells for the growth of hematopoietic cells. See also references cited therein. Glanville, et al., Nature 292:267-269, (1981), describe the mouse metallothionein-I gene. Wong, et al., Science 228:810-815, (1985), describe human GM-CSF. Lemischka, et al., Cell 45:917-927, (1986), describe retrovirus-mediated gene transfer as a marker for hematopoietic stem cells and the tracking of the fate of these cells after transplantation. Yang, et al., Cell 47:3-10, (1986), describe human IL-3. Chen and Okayama, Mol. Cell. Biol. 7:2745-2752, (1987), describe transformation of mammalian cells by plasmid DNA. Greaves, et al., Cell 56:979-986, (1989), describe the human CD2 gene.

Dexter et al, Long-Term Bone Marrow Culture, 1984, Alan R. Liss, Inc., pages 57-96 disclose the long- term culture hematopoietic cells. US-A-4810643, and US-A-4847201 disclose specific growth factors.

### SUMMARY OF THE INVENTION

Methods are provided employing reactors and compositions which allow for the efficient proliferation of hematopoietic cells in culture, particularly cells at an early stage in maturation, including stem cells. The methods employ transformed stromal cells which provide for constitutive or inducible production of growth factors, which cells are physically separated to allow for easy separation of hematopoietic cells. By providing for continuous perfusion, high densities and yields of viable hematopoietic cells may be achieved. The reactor employs a protein surface for the stromal cells and for maintaining separation of stromal cells and hematopoietic cells.

### BRIEF DESCRIPTION OF THE DRAWINGS

Figure 1 is a schematic view of a perfusion chamber; and
Figure 2 is a schematic representation and flow diagram of the perfusion medium pathway.

### DESCRIPTION OF THE SPECIFIC EMBODIMENTS

Methods are provided for the growth of hematopoietic cells in culture, employing transformed fibroblast cells for providing growth factors, proteinaceous components added to the mixtures of the transformed cells and the hematopoietic cells and substantially continuous perfusion to maintain an effective growth environment. The description of the method therefore may be divided into descriptions of the perfusion conditions, the reactor and its internal structure, and the transformed fibroblasts.

The reactor comprises a vessel which may be of any convenient shape which allows for the necessary cell distribution, introduction of nutrients and oxygen, permits removal of waste metabolic products, and harvesting of cells. The reactor should provide for conditions which substantially mimic bone perfusion. In vivo, about 0.08 ml of serum per ml of bone marrow per minute is perfused. This translates into about 3 ml of serum per 10⁶ cells per day. The media will therefore be changed on the average at least 50%, preferably at least 100%, in any 24 hour period, so as to maintain a level of metabolic products which is not growth limiting. The rate of change will generally be from about 5 to 10 ml of perfusion medium per 10⁶ cells per day, empirically mimicking in vivo perfusion rates.

Various media may be employed for the growth of hematopoietic and stromal cells. Illustrative media include MEM (Minimum Essential Medium), IMDM (Iscove's Modified Dulbecco's Medium), RPMI (Rosewell Park Memorial Institute), and may be supplemented by combinations of 5-20% fetal calf serum, 5-20% calf serum, and 5-15% horse serum, or serum free media supplemented with PDGF, EGF, FGF or other growth factors to stimulate stromal cells or stem cells. To supplement the growth factors provided by the transformed fibroblasts, additional growth factors may be included in the perfusion medium, particularly where dedicated cells of a particular lineage are desired. Among the growth factors which may be included in the perfusion medium, either by stromal cell excretion or addition, are GM-CSF, G-CSF, or M-CSF, interleukins 1-7, particularly 1, 3, 6 and 7, TGF-α or β, erythropoietin, or the like, particularly human factors. It is understood that one or more, preferably at least two of the growth factors will be provided by secretion from transformed cells, which will be present in an amount sufficient to maintain the desired level of the growth factors in the perfusion medium.

Conveniently, in the reactor physiologic temperature will be employed, namely 37°C, although lower temperatures may also be employed, including 33°, usually not being below 25°C. Humidity will generally be about 100%, where the air will contain about 5% carbon dioxide. The perfusion medium may be oxygenated external to the reactor or internal to the reactor, various means being provided for internal oxygenation. Internal oxygenation may be achieved with hollow fibers, porous sintered disks, silicone tubing or other membranes of suitable porosity and hydrophobicity. The nutrient level and metabolic product level will normally be maintained in a relatively narrow range. Glucose level will usually be in the range of about 5 to 20 mM, usually about 10 to 20 mM, lactate concentration will usually be maintained below about 35 mM and may be allowed to be over 20 mM. Glutamine concentration will generally be maintained in the range of about 1 to 3 mM, usually 1.5 to 2.5 mM, while ammonia concentration will usually be maintained below about 2.5 mM, preferably below about 2.0 mM.

The flow of fluid may be by gravity, by a pump, or other means, where the flow may be in any direction or a multiplicity of directions, depending upon the nature of the packing in the reactor. Desirably, laminar flow may be employed where the flow may be substantially horizontal across the reactor or vertical flow may be employed, where the flow is from the bottom to the top of the reactor or visa-versa.

A variety of packings may be used in the reactor to provide for adherent growth of the cells, while maintaining some physical separation between the stromal cells and the hematopoietic cells, and while allowing for some contact or close juxtaposition between the stromal cells and the hematopoietic cells. In this way, the factors secreted by the stromal cells may be readily taken up by the hematopoietic cells to encourage their proliferation and, as appropriate, differentiation and maturation.

The protein matrix to support the cells may take the form of shredded collagen particles, e.g., sponges or porous collagen beads, sponges or beads composed of extra-cellular bone matrix protein from bone marrow, or protein coated membranes, where the protein may be collagen, fibronectin, hemectin, RGDS peptide, mixed bone marrow matrix protein, or the like. Pore sizes of membranes will generally range from about 1 to 5 µ to allow for interaction between the different cell types, while still retaining physical separation.

Membranes may be employed, which will be protein coated. Various membrane materials may be employed such as polypropylene, polyethylene, polycarbonate, polysulfonate, etc. Various proteins may be employed, particularly collagen or the other proteins which were indicated previously. The membrane should have sufficiently small pores, that the transformed cells may not pass through the membranes, but may grow and form a confluent layer on one side of the membrane and extend portions of the cell membrane into the pores. Generally the pores will be in the range of about 1 to 5 µm. In this manner, the hematopoietic stem cells may grow on the opposite side of the membrane and interact with the transformed cells, whereby factors may be transferred directly from the transformed cells to the hematopoietic progenitor cells. The progenitor cells, the stem cells, are able to attach to the intruded cytoplasmic projections which have passed into the pores. Hematopoietic differentiation from the stem cells occurs on one side of the membrane and differentiated progeny are unable to squeeze back through the pores, which are already largely occupied by cytoplasmic projections from the fibroblasts. As hematopoietic cells mature and differentiate, they will be released from the membrane and into the nutrient medium.

The reactor may be packed with the various particles in a central portion of the reactor to define a central chamber, which will be separated from an upper chamber and a lower chamber. Alternatively, one or a plurality of membranes may be introduced, where two membranes will define a region associated with either the stromal cells or the hematopoietic cells, where the regions will alternate between stromal and hematopoietic cells. In this way, one may provide for differential perfusion rates between the chambers of the hematopoietic cells and the stromal cells. The medium exchange rate will generally fall within the ranges indicated above.

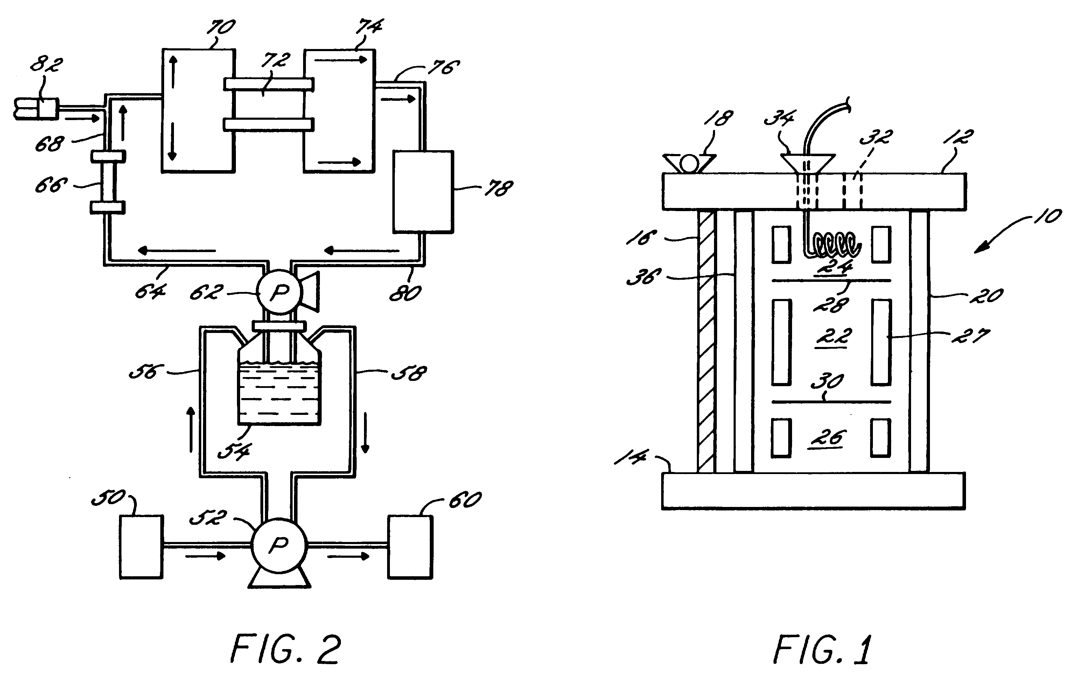

Figure 1 is a schematic view of a perfusion chamber. Reactor 10 with cover plate 12 and floor plate 14 are joined by bolts 16, held in position by wing nuts 18. Three bolts are employed, so as to avoid warping. The chamber 20 has three sections, the middle section 22 containing the support matrix for the stromal cells, the bed of stromal cells, and the bone marrow cells. The central section 22 is separated from the top section 24 and the bottom section 26 by membranes or mesh 28 and 30 respectively. Conveniently, polysulfonate membrane may be employed or a stainless steel mesh, whose mesh size is small enough so that cells are contained within the central section of the chamber. The separating interphase may be placed in the chamber using an inner cylinder 27 which is sectioned to provide the separating membrane mechanical support. The top section 24 and the bottom section 26 need not be identical and will have tubing or membranes across which liquid media and gases are exchanged. The gases are exchanged across a hydrophobic, e.g., silicone, tube whose length (and thereby gas/liquid contact area) may be varied to allow for sufficient gas fluxes to support the needs of the cell population that is metabolizing in the central section. The media can be pumped or withdrawn directly from the top or bottom sections through port 32 and may be fed through delivery tube 34.

If desired, the top and bottom sections may be eliminated by using an external oxygenator. In this situation, the separating membrane is held in place under the glass cylinder 36 which fits into cylindrical groove plates 12 and 14 and the area inside of the cylindrical groove is indented to allow for good flow distribution across the membrane. This geometry allows the fluid from the finite number of inlet ports to mix and for radial pressure to equilibrate, leading to a uniform liquid flow across the separating membrane. This setup is suitable for chambers which have relatively few cells, so that oxygenation does not become limiting.

In Figure 2 is depicted a schematic representation of the loop that connects the perfusion chamber to the side media reservoir, oxygenator, sensor chamber, and sample/injection ports.

An external fresh media source 50 is pumped by means of pump 52 to a media reservoir through line 56 and spent media is withdrawn through line 58 from reservoir 54 by means of pump 52 to the spent media container 60 for further processing. A second pump 62 pumps media from the media reservoir 52 through line 64 through a hollow fiber oxygenator 66. The media is directed through line 68 to the first chamber of bioreactor 70. As appropriate, a means for injection of media component 82 is provided, for introducing the component into line 68 for transport by the media into the first chamber of bioreactor 70. The component may be test components, additional factors, or the like. The media from bioreactor 70 is directed through central chamber 72 into the second chamber 74 of the bioreactor. From there the media is directed by line 76 to in-line sensors 78 for detecting the change in composition of the media.

For example, it is desirable that the glutamine:glucose ratio be in the range of about 1:5-8, depending on the cell lines used; for instance, preferably 1:8 for transfected 3T3 cells. Furthermore, ammonium concentrations will preferably be below about 2.0 mM and lactate concentrations are preferably less than about 40 mM. By monitoring the effluent from the bioreactor, the media introduced into the bioreactor may be modified, oxygen partial pressure may be changed, gas flow rate may be altered, various components may be augmented, or the rate of perfusion may be slowed or increased.

From the sensors 78, the media is directed through line 80 by means of pump 62 to the reservoir 54.

By means of the flow path described above, the media in the side reservoir is slowly exchanged using a separate pump. This organization allows for separate control of the media exchange rate (the outer pump) and the flow rate through the oxygenator and perfusion chamber. The former is used to control the longer term change in the media composition and perfusion, while the latter may be used to control the dissolved oxygen tension and flow patterns in the chamber. The use of a small mesh biocompatible membrane allows for plug (piston) flow in the chamber and thus allows the precise control of delivery of growth factors and other special compounds that one may wish to introduce to the hematopoietic cells and stromal cells in very precise amounts.

After autoclaving the chamber and components of the loop, the reactor is assembled in a sterile environment. The media may be circulated through the side loop and chamber for a few days while signs of contamination are monitored. If sterile assembly is accomplished, the central section of the chamber is inoculated with either the extra-cellular matrix alone or a pre-inoculated extra-cellular matrix support that contains the stromal cells. The stromal cells are then either: 1) kept in the chamber for a period of a few days while their metabolic performance and/or growth factor responsiveness is monitored and if results are satisfactory, the bone marrow is inoculated; or 2) immediately seeded with bone marrow. In either case, the cell layer is kept at the bottom of the central section of the perfusion chamber. The cells lay down additional extra-cellular matrix and the cell layer adheres to the separating membrane. At this time, the chamber may be inverted and the cell layer may then be located at the ceiling of the central section. In this configuration, the maturing cells will settle on the bottom of the central chamber as they lose their adherence to the stromal layer. This feature is important to prevent the damage caused by mature cells to the stromal layer and/or the less mature hematopoietic cells. This feature also makes the continuous removal of mature cells easier.

These cells are harvested by withdrawing the cells by syringe, or by continuously allowing the cells to flow out of the chamber, by the pressure of the perfused medium, through the exit tubing.

The stromal cells will, for the most part, be fibroblasts transformed with one or more genes providing for desired hematopoietic growth factors. The same or different cells may be transfected with the genes, depending upon the particular selection of host cells, the same or different cells may be used for a plurality of genes.

A wide variety of normal cells or stable lines may be employed. However, it is found that not all cell strains are permissible, since transformation of some cell lines may result in the overgrowth of the cells. Desirably, the cells which are employed will not be neoplastic, but rather require adherence to a support. The mammalian cells need not be human, nor even primate. A variety of nontransformed cells may be included in the adherent cell layer as well, including normal human bone marrow adherent cells, normal human spleen adherent cells, and normal human thymic epithelium.

Methods for transforming mammalian cells, including fibroblasts, are well known and there is an extensive literature of which only a few references have been previously given. The constructs may employ the naturally occurring transcriptional initiation regulatory region, comprising the promoter and, as appropriate the enhancer, or a different transcriptional initiation region may be involved, which may be inducible or constitutive.

A large number of transcriptional initiation regions are available which are inducible or constitutive, may be associated with a naturally occurring enhancer, or an enhancer may be provided, may be induced only in a particular cell type, or may be functional in a plurality or all cell types. The transcriptional initiation region may be derived from a virus, a naturally occurring gene, may be synthesized, or combinations thereof.

Promoters which are available and have found use include the chromosomal promoters, such as the mouse or human metallothionein-I or II promoters, β-actin promoter, etc., or viral promoters, such as SV40 early gene promoters, CMV promoter, adenovirus promoters, promoters associated with LTRs of retroviruses, etc. These promoters are available and may be readily inserted into appropriate vectors which comprise polylinkers for insertion of the transcriptional initiation region as well as the gene of interest. In other instances, expression vectors are available which provide for a polylinker between a transcriptional initiation region and a transcriptional termination region, also providing for the various signals associated with the processing of the messenger for translation, i.e., the cap site and the polyadenylation signal. The construction of the expression cassette comprising the regulatory regions and the structural gene may employ one or more of restriction enzymes, adaptors, polylinkers, in vitro mutagenesis, primer repair, resection, or the like.

The expression cassette will usually be part of a vector which will include a marker and one or more replication systems. The marker will allow for detection and/or selection of cells into which the expression cassette and marker have been introduced. Various markers may be employed, particularly markers which provide for resistance to a toxin, particularly an antibiotic. Preferably, gentamycin resistance is employed, which provides resistance to G418 for a mammalian cell host. The replication systems may comprise a prokaryotic replication system, which will allow for cloning during the various stages of bringing together the individual components of the expression cassette. The other replication system may be used for maintenance of an episomal element in the host cell, although for the most part the replication system will be selected so as to allow for integration of the expression cassette into a chromosome of the host.

The introduction of the expression cassette into the host may employ any of the commonly employed techniques, including transformation with calcium precipitated DNA, transfection, infection, electroporation, ballistic particles, or the like. Once the host cells have been transformed, they may be amplified in an appropriate nutrient medium having a selective agent, to select for those cells which comprise the marker. Surviving cells may then be amplified and used.

Host cells which may be employed include African green monkey cell line CVl, mouse cells NIH-3T3, normal human bone marrow fibroblasts, human spleen fibroblasts, normal mouse bone marrow fibroblasts, and normal mouse spleen fibroblasts. It should be noted that in some instances, depending upon the choice of vector and cell line, the cells may become neoplastic. It is important that the resulting transformed cells be capable of adherence, whereby the transformed cells maintain binding to a support, such as protein sponges, protein coated membranes, or the like.

Once the vector for expressing the appropriate growth factors has been constructed, it may be used to transform the cells by any convenient means. The resulting transformed cells may then be used to seed the supports, which have already been described. These supports may be introduced into the reactor or may be present at the time of seeding in the reactor. The cells will be allowed to grow for sufficient time to ensure that the cells are viable and are capable of producing the desired growth factors.

The reactor may then be seeded as appropriate with the hematopoietic cells. The hematopoietic cells may include substantially pure stem cells, a mixture of hematopoietic cells substantially free of mature hematopoietic cells of one or more lineages, or a mixture comprising all or substantially all of the various lineages of the hematopoietic system, at various stages of their maturation.

The cells are allowed to grow with substantially continuous perfusion through the reactor and monitoring of the various nutrients and factors involved. For the most part, the primary factors will be provided by the stromal cells, so that a steady state concentration of growth factors will normally be achieved. Since conditioned supernatants are found to be effective in the growth of the hematopoietic cells, one can provide for a ratio of stromal cells to hematopoietic cells which will maintain the growth factor at an appropriate concentration level in the reactor.

Transfected stroma can provide for the introduction of genes into human stem cells. In mice, retroviral mediated gene transfer into stem cells is made possible by pretreating mice with 5-fluoro uracil (5-FU) and then growing the harvested bone marrow cells in WEHI (Walter & Eliza Hall Institute) conditioned media, which contains IL-3 and GM-CSF (Lemischka, Cell 45:917, 1986). The artificial stroma, grown with a retroviral packaging cell line secreting a retroviral vector of interest, may be used to efficiently introduce genes into human stem cells. For example, human T-cells could be made resistant to HIV infection by infecting stem cells with the retroviral vector containing an HIV antisense sequence under control of a CD2 regulatory sequence (Greaves, Cell 56:979-986, 1989) which would allow for tissue specific expression in T-cells. There would be a factor provided by the retroviral packaging cell line essential for replication of the retrovirus; this factor would be absent in the hematopoietic target cells. Once the virus was transferred to the hematopoietic target cells, it would no longer be able to replicate.

The following examples are offered by way of illustration and not by way of limitation.

### EXPERIMENTAL

### I. Formation of Transformants

The growth factor human GM-CSF (Wong, Science, 228:810-815, (1985)) was inserted into a eukaryotic expression vector. The hGM-CSF cDNA (EcoRI to AhaIII, approximately 700 bp fragment) was cloned into an EcoRI to PstI fragment of pSP65. (Melton, Nucl. Acids Res. 2:7035-7056 (1984)). The resulting plasmid was pSP65GM-CSF. The mouse metallothionein promoter (Glanville, Nature, 292:267-269, (1981)) was digested with EcoRI and BglII and the approximately 2 kb fragment containing the promoter was inserted into the EcoRI to BamHI fragment of pSP65 to make p65MT. The plasmid pMT GM-CSF was then constructed by digesting pSP65GM-CSF with EcoRI, filling in the overhang with the Klenow fragment of DNA polymerase I and then digesting the resulting linearized DNA with HindIII to isolate the 700 bp fragment comprising the coding region of GM-CSF. This fragment was subcloned into the SalI filled/HindIII site of p65MT. The 2.7 kb fragment comprising the metallothionein promoter and the GM-CSF coding region was then isolated and placed into pSV2neo (Southern and Berg, J. Mol. Appl. Genet 1:327 (1982)) from which the SV-40 promoter was removed. This results in the SV-40 poly A signal downstream of the GM-CSF coding sequence.

The neomycin resistant gene, which confers resistance to the antibiotic gentamycin (G418) was taken from pSV2neo by isolating the approximately 3 kb PvuII to EcoRI fragment and placing EcoRI linkers onto the PvuII site. The neo resistance gene with EcoRI ends was subcloned into the EcoRI site of the GM-CSF expression plasmid to create the plasmid MTGM-CSFneo.

The plasmid MTGM-CSFneo alone and as a cotransfection with the plasmid (Yang, Cell 47:3-10, 1986) encoding the gibbon ape IL-3 gene under the control of the SV-40 promoter and poly A site, were transfected by electroporation of linearized DNA into the African green monkey cell line CV1 and the mouse cell line NIH 3T3 cells. Transformants were selected by selection in media containing 500 mg/ml of G418, isolated, and screened for production of GM-CSF or IL-3 by bioassay of supernatants using AML-193 cells (Adams, et al., Leukemia 3:314 (1989)). Several of the positive lines were then employed as stroma for human bone marrow cells in Dexter culture.

In addition, normal mouse bone marrow cells were transfected with the above plasmids using the calcium/phosphate method of Okayama (Chen, Mol. Cell. Biol. 7:2745-2752, 1987) and were found to efficiently express the introduced genes.

GM-CSF and IL-3 secretion by the transfected fibroblasts was investigated. Serum free 72 hour culture supernatants were obtained from the NIH-3T3 cells and assayed for hGF secretion by ³H uptake on target cells inhibitable by neutralizing rabbit anti-GM-CSF or anti-IL-3 antibodies. Proliferation induced by 20 mg/ml GM-CSF was set as 100 units GM-CSF and that induced by 10 ng/ml IL-3 was set as 100 units IL-3. The co-transfected cells produced about 35 units/ml of GM-CSF and about 57 units/ml of IL-3.

### II. Perfusion Chamber

The perfusion chamber is a glass cylinder with Delrin caps to allow for autoclaving without deformation and biocompatability. The caps have cylindrical groves into which the glass cylinder fits. At the bottom of the grove an O-ring is placed to seal the lumen of the chamber. The caps have several holes into which Luer (Luer Lok) fittings are provided into which media and gas delivery lines are put as well as an extended tube into the central section of the chamber to sample adherent and/or non-adherent cells. The caps are attached with three long bolts, spaced 120°, placed outside the glass cylinder; wing nuts and washers are used to tighten the assembly.

The chamber is hooked to a side reservoir. The loop contains a pump, a chamber of on-line sensors, oxygenator, and sample and injection ports in addition to the side media reservoir. The media in the side reservoir is then slowly exchanged using a separate pump. This configuration allows for separate control of the media exchange rate and the flow rate through the oxygenator and perfusion chamber. The former is used to control the longer term change in the media composition and perfusion, while the latter may be used to control the dissolved oxygen tension and flow patterns in the chamber. The use of a small mesh polysulfonate membrane allows for plug flow in the chamber and the precise control of delivery of growth factors and other special compounds which one may wish to introduce into the bioreactor in very precise amounts.

The transfected stromal cells are seeded either over a bed of shredded collagen sponge or the stromal cells are placed on one side of a 5µ porous polycarbonate filter precoated with collagen and the stromal cells allowed to adhere to the filter over a number of hours. The cells are allowed to grow in an appropriate nutrient medium until the cells become confluent on one side while sending cytoplasmic projections through the pores. Bone marrow cells are then seeded on the other side of the membrane and the stem cells attach to the intruded cytoplasmic projections which have passed through the pores.

After autoclaving the chamber and components of the loop, the reactor is assembled in a sterile environment. The media is then circulated through the side loop and chamber for a few days while signs of contamination are monitored. The central section of the bioreactor is then inoculated with either the extra-cellular matrix alone or a pre-inoculated extra-cellular matrix support that contains the stromal cells. The stromal cells may then be kept in the chamber for a period of a few days while their metabolic performance and/or growth factor responsiveness is monitored and if results are satisfactory, the bone marrow is inoculated or immediately seeded with bone marrow. In either case, the cell layer is kept at the bottom of the central section of the perfusion chamber.

The cells lay down additional extra-cellular matrix and the cell layer adheres to the support. Where the membrane is used, the chamber may be inverted and the cell layer is then located at the ceiling of the central section. In this configuration, the maturing cells settle on the bottom of the central chamber as they loose their adherence to the stromal layer. The non-adherent cells are then harvested by constant cell flow, driven by the medium perfusion pressure, into the exit tubing.

In a typical run, the chamber was inoculated with NIH-3T3 cells on day one on shredded collagen sponge support. For the first 40 days perfusion rates and other operating variables were adjusted. At day 40 a reasonable steady state was achieved which was maintained for about 20 days. On day 64 the chamber was seeded with 33 x 10⁶ human bone marrow cells. For the first 10 days the harvested cell count decreased until it settled in a steady state of about 7-8 x 10⁵ cells produced every three days. Flow cytometric analysis showed that a constant fraction, about 20% of the harvested cells were HLA-DR positive. On day 90 a pump failure was experienced and the pH dropped below 6.9 overnight. When the perfusion rate was restored the production of non-adherent cells recovered and was approaching the previous steady state production rate when a bacterial contamination occurred. At this point, the study was terminated.

The above results demonstrated that a perfusion chamber is capable of performing ex vivo hematopoiesis, hematopoiesis may be restored ex vivo after a pH drop, the glucose concentration data showed that the hematopoietic cells grow primarily aerobically on glucose, since the glucose concentration drops after inoculation without increasing the lactate concentration indicating that oxygenation is limiting. The glucose/lactate (anaerobic) metabolism appears to be primarily due to the NIH-3T3 stromal bed. Similarly, the glutamine and ammonia concentrations reach pre-inoculum levels once the hematopoietic cell number levels off, implying that the glutamine consumption by the bone marrow cells is much less than that of the stromal bed.

### III. Monitoring of Metabolic Products

The consumption and formation rates of glucose and lactate as well as glutamine and ammonia were determined for transfected NIH-3T3 cells. (The medium was IMDM plus 20 % FCS -Fetal Calf Serum-). Increased glucose consumption was only observed for daily fed T-flasks, where a all less frequently fed cultures follow the same slowly diminishing glucose uptake rate pattern. Cultures that were exchanged 50% daily were switched to the 100% daily exchange schedule on day 18, which resulted in an immediate increase in glucose consumption following the same trend as that observed for cultures exchanged 100% daily from day one. Lactate production rates follow a similar pattern, as the lactate yield on glucose is essentially a constant (0.9 lactate/glucose; indicating a largely anaerobic stromal metabolism).

The glutamine and ammonia concentrations show a pattern analogous to the glucose/lactate metabolism. Using values corrected for chemical decomposition of glutamine at 37°C, the glutamine consumption rate versus the glucose consumption rate shows relative uptake rates are constant, about 1:8 glutamine: glucose. The predicted optimum ratio varies with oxygen uptake rate - the ratio drops with increasing optimum uptake rate.

Analogous conclusions were supported by glucose/lactate metabolic data derived from normal bone marrow stromal fibroblasts. Under conditions of infrequent medium exchange the cultures were primarily anaerobic, with high steady state levels of lactate rapidly achieved and maintained. With more frequent medium exchange, the cell metabolism became more rapid, with increased glucose consumption and lactate production. No detectable consumption of glutamine was observed after correcting the data for spontaneous chemical decomposition. For both 3T3 cells and normal human bone marrow cells, the cells continue to divide and crowd when the serum/media exchange rate was above what appears to be a critical replacement schedule.

To further ascertain the relative importance of perfusion rate of serum versus that of nutrients, the following experiments were performed: 1) one set of T-flasks with 20% serum containing media exchanged daily; 2) two sets of T-flasks, one with 20% serum and the media exchanged every other day and one with 10% serum with the media exchanged daily; 3) two sets of T-flasks, one with 10% serum and the media exchanged every other day, one with 5% serum with the media exchanged daily; 4) two sets of T-flasks, one with 5% serum and the media exchanged every other day and one with 2.5% serum with the media exchanged daily. The serum exchange rate is the same within each group while the exchange rate of the nutrient containing media varies. The results from these experiments show that it is the exchange rate of the serum that is critical. While for the experiment 1) glucose consumption increased and by day four had substantially flattened out to a rate of about 9.5 mmoles/per day, in all of the other cases, the glucose consumption started below the original glucose consumption of Group I and dropped off in a substantially linear manner regardless of whether twice the amount of serum was used and changed every other day or half the amount of serum was used and the media changed every day. This supports the need for a critical perfusion rate of serum or one or more serum components that influence the metabolic growth behavior of the stromal cells.

It is evident from the above results, that one may grow hematopoietic cells in a bioreactor in an efficient manner. Stromal cells can be provided from homologous or heterologous sources, where the stromal cells have been transfected with genes to provide for the important growth factors. In this manner, serum need not be added to the media to support the growth of the cells. By providing for stromal cells which adhere to a support in a manner which allows for separation of hematopoietic cells from the stromal cells, the hematopoietic cells may be continuously harvested for use. By appropriate choice of combinations of growth factors, specific lineages of hematopoietic cells may be grown. In addition, if desired, the stromal cells may provide for a reservoir of transfecting viruses for the introduction of genes into the hematopoietic cells.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims.

The object of the invention is defined in the claims.

## Claims

1. A method of growing human stem cells in culture, said method comprising:
inoculating a reactor vessel comprising stromal cells with human hematopoietic cells comprising human stem cells, wherein at least a portion of said stromal cells are transformed fibroblast cells capable of excreting at least one growth factor which directs the proliferation and/or differentiation of said human stem cells;
substantially continuously perfusing said cells in said reactor with a nutrient medium comprising any additional growth factors necessary for the proliferation and/or differentiation of said human stem cells, said perfusing being at a rate sufficient to obtain proliferation of said human stem cells, while removing metabolic products, replenishing depleted nutrients, and maintaining said reactor under physiologically acceptable conditions; and
harvesting cells from said reactor.

2. A method according to Claim 1, wherein said cells in said reactor are substantially continuously perfused at a rate of at least 50% daily medium replacement.

3. A method according to Claim 1, wherein said cells in said reactor are substantially continuously perfused at a rate of from about 5 to 10 ml of said nutrient medium per 10⁶ cells per day.

4. A method according to Claim 1, wherein said human hematopoietic cells comprise human bone marrow cells.

5. A method according to Claim 1, wherein said perfusing provides a glucose concentration in the range of about 5-20 mM and a glutamine concentration in the range of about 1-3 mM, while the lactate concentration is maintained below about 35 mM and the ammonia concentration is maintained below about 2.5 mM.

6. A method according to claim 1, wherein said perfusing is at a rate sufficient to obtain proliferation and differentiation of said human stem cells.

7. A method according to Claim 1, wherein said stromal cells excrete at least one of a colony stimulating factor or an interleukin.

8. A method according to Claim 7, wherein said colony stimulating factor is human GM-CSF and said interleukin is human IL-3.

9. A method according to Claim 1, wherein said stromal cells in said reactor vessel are adherent to a protein coated membrane or a protein sponge.

10. A method according to Claim 1, wherein said growth factor is GM-CSF, G-CSF, M-CSF, one of interleukins 1-7, TGF-α or -β, or erythropoietin.

11. A method of growing human stem cells in culture, said method comprising:
inoculating a reactor vessel comprising heterologous stromal cells adherent to one side of a protein membrane substrate with pores in the range of about 1-5 µm with human hematopoietic cells comprising human stem cells, said inoculation being on the opposite side of said membrane from said heterologous stromal cells, wherein at least a portion of said stromal cells are transformed fibroblast cells capable of adhering to a protein surface and capable of excreting at least one colony stimulating factor or interleukin which directs the proliferation and/or differentiation of said human stem cells;
substantially continuously perfusing said cells in said reactor with a nutrient medium comprising any additional growth factors necessary for the proliferation and/or differentiation of said human stem cells, said perfusing being at a rate sufficient to obtain proliferation of said human stem cells, while removing metabolic products, replenishing depleted nutrients, and maintaining said reactor under physiologically acceptable conditions; and
harvesting cells from said reactor.

12. A method according to Claim 11, wherein said perfusing is at a rate sufficient to obtain proliferation and differentiation of said human stem cells.

13. A method according to Claim 11, wherein said human hematopoietic cells are human bone marrow cells.

14. A method according to Claim 11, wherein said perfusing provides a glucose concentration in the range of about 5-20 mM and a glutamine concentration in the range of about 1-3 mM, while the lactate concentration is maintained below about 35 mM and the ammonia concentration is maintained below about 2.5 mM.

15. A bioreactor, comprising:
a reactor chamber comprising stromal cells and human hematopoietic cells comprising human stem cells, wherein at least a portion of said stromal cells are transformed fibroblast cells capable of excreting at least one growth factor which directs the proliferation and/or differentiation of said human stem cells;
means for effectuating the substantial continuous perfusion of said cells in said reactor with a nutrient medium, said nutrient medium comprising any additional growth factors necessary for the proliferation and/or differentiation of said human stem cells, and said perfusing being at a rate sufficient to obtain proliferation of said human stem cells in said reactor chamber, said means comprising a first means for introducing into and removing from said reactor chamber a nutrient medium permitting removal of metabolic products, replenishing depleted nutrients, and maintaining said reactor under physiologically acceptable conditions; said means further comprising a second means for monitoring the effluent from said reactor chamber.

16. A bioreactor according to Claim 15, wherein said stromal cells are adherent to a protein substrate, said protein substrate being a protein-coated membrane with pores of a size in the range of about 1-5 µm with said stromal cells being adherent to one side of said membrane and said human hematopoietic cells being present on the opposite side.

17. A bioreactor according to Claim 15, wherein said first means for introducing and removing said nutrient medium comprises:
a media reservoir for storing media;
a means for transporting fresh media into said reservoir and removing partially spent media from said reservoir;
a means for transporting media from said reservoir to said bioreactor and from said bioreactor to said reservoir;
a means for oxygenating said media prior to introducing into said bioreactor; and
means for monitoring the composition of said media in said bioreactor.

## Patentansprüche

1. Ein Verfahren zum Wachstum menschlicher Stammzellen in Kultur, wobei das Verfahren umfaßt:
Beimpfen eines Reaktorgefäßes, enthaltend Stromazellen mit menschlichen hämopoetischen Zellen, umfassend menschliche Stammzellen, wobei wenigstens ein Teil der Stromazellen transformierte Fibroblastzellen mit Befähigung zur Exkretion von wenigstens einem Wachstumsfaktor sind, der die Proliferation und/oder Differenzierung der menschlichen Stammzellen lenkt;
im wesentlichen kontinuierliches Durchströmen der Zellen im Reaktor mit einem Nährmedium, enthaltend jedwede zusätzliche Wachstumsfaktoren, die für die Proliferation und/oder Differenzierung der menschlichen Stammzellen notwendig sind, wobei das Durchströmen bei einer Rate erfolgt, die ausreicht, um Proliferation der menschlichen Stammzellen zu erhalten, bei gleichzeitigem Entfernen von Stoffwechselprodukten, Wiederauffüllen verbrauchter Nährstoffe und Aufrechterhalten des Reaktors unter physiologisch annehmbaren Bedingungen und
Ernten von Zellen vom Reaktor.

2. Ein Verfahren nach Anspruch 1, bei dem die Zellen im Reaktor im wesentlichen kontinuierlich bei einer Rate von wenigstens 50% des täglichen Mediumersatzes durchströmt werden.

3. Ein Verfahren nach Anspruch 1, bei dem die Zellen im Reaktor im wesentlichen kontinuierlich bei einer Rate von ungefähr 5 bis 10 ml des Nährmediums pro 10⁶ Zellen pro Tag durchströmt werden.

4. Ein Verfahren nach Anspruch 1, bei dem die menschlichen hämopoetischen Zellen menschliche Knochenmarkszellen umfassen.

5. Ein Verfahren nach Anspruch 1, bei dem das Durchströmen eine Glukosekonzentration im Bereich von etwa 5 bis 20 mM und eine Glutaminkonzentration im Bereich von etwa 1 bis 3 mM bereitstellt, während die Lactatkonzentration unterhalb etwa 35 mM und die Ammoniakkonzentration unterhalb etwa 2,5 mM aufrechterhalten wird.

6. Ein Verfahren nach Anspruch 1, bei dem das Durchströmen bei einer Rate erfolgt, die ausreicht, um Proliferation und Differenzierung der menschlichen Stammzellen zu erhalten.

7. Ein Verfahren nach Anspruch 1, bei dem die Stromazellen wenigstens einen eine Kolonie-stimulierenden Faktor oder ein Interleukin exkretieren.

8. Ein Verfahren nach Anspruch 7, bei dem der Kolonie-stimulierende Faktor menschliches GM-CSF und das Interleukin menschliches IL-3 ist.

9. Ein Verfahren nach Anspruch 1, bei dem die Stromazellen im Reaktorgefäß an eine Protein überzogene Membran oder einen Proteinschwamm geheftet sind.

10. Ein Verfahren nach Anspruch 1, bei dem der Wachstumsfaktor GM-CSF, G-CSF, M-CSF, eines der Interleukine 1-7, TGF-α oder -β oder Erythropoietin ist.

11. Ein Verfahren zum Wachstum menschlicher Stammzellen in Kultur, bei dem das Verfahren umfaßt:
Beimpfen eines Reaktorgefäßes, enthaltend heterologe Stromazellen, die an einer Seite eines Proteinmembransubstrates mit Poren im Bereich von etwa 1 bis 5 µm geheftet sind, mit menschlichen hämopoetischen Zellen, umfassend menschliche Stammzellen, wobei die Beimpfung auf der Gegenseite der Membran von den heterologen Stromazellen erfolgt, wobei wenigstens ein Teil der Stromazellen transformierte Fibroblastzellen sind, mit Befähigung zur Anheftung an eine Proteinoberfläche und zur Exkretion wenigstens eines Kolonie-stimulierenden Faktors oder Interleukins, was die Proliferation und/oder Differenzierung der menschlichen Stammzellen lenkt;
im wesentlichen kontinuierliches Durchströmen der Zellen im Reaktor mit einem Nährmedium enthaltend jedwede zusätzliche Wachstumsfaktoren, die für die Proliferation und/oder Differenzierung der menschlichen Stammzellen notwendig sind, wobei das Durchströmen bei einer Rate erfolgt, die ausreicht, um Proliferation der menschlichen Stammzellen zu erhalten, bei gleichzeitigem Entfernen von Stoffwechselprodukten, Wiederauffüllen verbrauchter Nährstoffe und Aufrechterhalten des Reaktors unter physiologisch annehmbaren Bedingungen; und
Ernten von Zellen vom Reaktor.

12. Ein Verfahren nach Anspruch 11, bei dem das Durchströmen bei einer Rate erfolgt, die ausreicht, um Proliferation und Differenzierung der menschlichen Stammzellen zu erhalten.

13. Ein Verfahren nach Anspruch 11, bei dem die menschlichen hämopoetischen Zellen menschliche Knochenmarkszellen sind.

14. Ein Verfahren nach Anspruch 11, bei dem das Durchströmen eine Glukosekonzentration im Bereich von etwa 5 bis 20 mM und eine Glutaminkonzentration im Bereich von etwa 1 bis 3 mM bereitstellt, während die Lactatkonzentration unterhalb etwa 35 mM und die Ammoniakkonzentration unterhalb etwa 2,5 mM aufrechterhalten wird.

15. Ein Bioreaktor, umfassend:
eine Reaktorkammer, enthaltend Stromazellen und menschliche hämopoetische Zellen, umfassend menschliche Stammzellen, worin wenigstens ein Teil der Stromazellen transformierte Fibroblastzellen mit Befähigung zur Exkretion von wenigstens einem Wachstumsfaktor sind, der die Proliferation und/oder Differenzierung der menschlichen Stammzellen lenkt;
Mittel zur Ausführung der wesentlichen kontinuierlichen Durchströmung der Zellen im Reaktor mit einem Nährmedium, wobei das Nährmedium jedwede zusätzliche Wachstumsfaktoren enthält, die für die Proliferation und/oder Differenzierung der menschlichen Stammzellen notwendig sind, und wobei das Durchströmen bei einer Rate erfolgt, die ausreicht, um Proliferation der menschlichen Stammzellen in der Reaktorkammer zu erhalten, wobei die Mittel ein erstes Mittel zur Einführung und zum Entfernen von Nährmedium in die bzw. von der Reaktorkammer umfaßt und Entfernung von Stoffwechselprodukten, Wiederauffüllen verbrauchter Nährstoffe und Aufrechterhalten des Reaktors unter physiologisch annehmbaren Bedingungen gestattet, wobei die Mittel weiterhin ein zweites Mittel zur Überwachung des Ausflusses aus der Reaktorkammer umfaßt.

16. Ein Bioreaktor gemäß Anspruch 15, bei dem die Stromazellen an ein Proteinsubstrat geheftet sind, wobei das Proteinsubstrat eine Protein-überzogene Membran mit Poren einer Größe im Bereich von etwa 1 bis 5 µm ist, mit den an einer Seite der Membran angehefteten Stromazellen und die an der Gegenseite vorhandenen menschlichen hämopoetischen Zellen.

17. Ein Bioreaktor nach Anspruch 15, bei dem das erste Mittel zur Einführung und zum Entfernen des Nährmediums umfaßt:
ein Mediumbehälter zur Mediumspeicherung;
ein Mittel für den Transport von frischem Medium in den Behälter und für das Entfernen von teilweise verbrauchtem Medium vom Behälter;
ein Mittel für den Transport von Medium vom Behälter zum Bioreaktor und vom Bioreaktor zum Behälter;
ein Mittel zur Sauerstoffanreicherung des Mediums vor dessen Einführung in den Bioreaktor; und
ein Mittel zur Überwachung der Zusammensetzung des Mediums im Bioreaktor.

## Revendications

1. Procédé de croissance de cellules souches humaines en culture, ledit procédé comprenant :
l'inoculation d'une cuve de réacteur comprenant des cellules du stroma avec des cellules hématopoïétiques humaines comprenant des cellules souches humaines, dans lequel au moins une partie desdites cellules du stroma sont des fibroblastes transformés, capables d'excréter au moins un facteur de croissance qui dirige la prolifération et/ou la différenciation desdites cellules souches humaines :
la perfusion essentiellement continue desdites cellules dans ledit réacteur avec un milieu nutritif comprenant tout les facteurs de croissance additionnels nécessaires à la prolifération et/ou à la différenciation desdites cellules souches humaines, ladite perfusion étant effectuée à un taux suffisant pour obtenir la prolifération desdites cellules souches humaines, tout en éliminant les produits métaboliques, en réapprovisionnant les substances nutritives épuisées et en maintenant ledit réacteur dans des conditions physiologiquement acceptables ; et
la récolte des cellules depuis ledit réacteur.

2. Procédé selon la revendication 1, dans lequel lesdites cellules dans ledit réacteur sont perfusées de façon essentiellement continue à un taux d'au moins 50% de remplacement journalier du milieu.

3. Procédé selon la revendication 1, dans lequel lesdites cellules dans ledit réacteur sont perfusées de façon essentiellement continue à un taux d'environ 5 à 10 ml dudit milieu nutritif par 10⁶ cellules par jour.

4. Procédé selon la revendication 1, dans lequel lesdites cellules hématopoïétiques humaines comprennent des cellules de moelle osseuse humaine.

5. Procédé selon la revendication 1, dans lequel ladite perfusion fournit une concentration en glucose comprise dans la gamme d'environ 5 à 20 mM et une concentration en glutamine dans la gamme d'environ 1 à 3 mM, tandis que la concentration de lactate est maintenue en dessous d'environ 35 mM et que la concentration d'ammoniac est maintenue en dessous d'environ 2,5 mM.

6. Procédé selon la revendication 1, dans lequel ladite perfusion est a un taux suffisant pour obtenir la prolifération et la différenciation desdites cellules souches humaines.

7. Procédé selon la revendication 1, dans lequel lesdites cellules du stroma excrètent au moins un facteur stimulant la colonie ou une interleukine.

8. Procédé selon la revendication 7, dans lequel ledit facteur stimulant la colonie est le GM-CSF humain et ladite interleukine est l'IL-3 humain.

9. Procédé selon la revendication 1, dans lequel lesdites cellules du stroma dans ladite cuve de réacteur adhérent à une membrane recouverte de protéines ou à une éponge de protéines.

10. Procédé selon la revendication 1, dans lequel ledit facteur de croissance est le GM-CSF, le G-CSF, le M-CSF, l'une des interleukines 1 à 7, le TGF-alpha ou bêta ou l'érythropoïétine.

11. Procédé de croissance de cellules souches humaines en culture, ledit procédé comprenant :
l'inoculation d'une cuve de réacteur comprenant des cellules hétérologues du stroma qui adhérent à un côté d'un substrat de membrane de protéines avec des pores compris dans la gamme d'environ 1 à 5 µm, avec des cellules hématopoïétiques humaines comprenant des cellules souches humaines, ladite inoculation étant effectuée sur le côté opposé de ladite membrane par rapport auxdites cellules hétérologues du stroma, dans lequel au moins une partie desdites cellules de stroma sont des fibroblastes transformés capables d'adhérer à une surface de protéines et capable d'excréter au moins un facteur stimulant la colonie ou une interleukine qui dirige la prolifération et/ou la différenciation desdites cellules souches humaines ;
la perfusion essentiellement continue desdites cellules dans ledit réacteur avec un milieu nutritif comprenant tout les facteurs de croissance additionnels nécessaires à la prolifération et/ou à la différenciation desdites cellules souches humaines, ladite perfusion étant effectuée à un taux suffisant pour obtenir la prolifération desdites cellules souches humaines, tout en éliminant les produits métaboliques, en réapprovisionnant les substances nutritives épuisées et en maintenant ledit réacteur dans des conditions physiologiquement acceptables ; et
la récolte des cellules depuis ledit réacteur.

12. Procédé selon la revendication 11, dans lequel ladite perfusion est effectuée à un taux suffisant pour obtenir la prolifération et la différentiation desdites cellules souches humaines.

13. Procédé selon la revendication 11, dans lequel lesdites cellules hématopoïétiques humaines sont des cellules de moelle osseuse humaine.

14. Procédé selon la revendication 11, dans lequel ladite perfusion fournit une concentration en glucose comprise dans la gamme d'environ 5 à 20 mM et une concentration en glutamine dans la gamme d'environ 1 à 3 mM, tandis que la concentration de lactate est maintenue en dessous d'environ 35 mM et que la concentration d'ammoniac est maintenue en dessous d'environ 2,5 mM.

15. Bioréacteur comprenant :
une chambre de réaction comprenant des cellules du stroma et des cellules hématopoïétiques humaines comprenant des cellules souches humaines, dans lequel au moins une partie desdites cellules du stroma sont des fibroblastes transformés, capables d'excréter au moins un facteur de croissance qui dirige la prolifération et/ou la différentiation desdites cellules souches humaines ;
des moyens pour effectuer la perfusion essentiellement continue desdites cellules dans ledit réacteur avec un milieu nutritif, ledit milieu nutritif comprenant tout les facteurs de croissance additionnels nécessaires à la prolifération et/ou à la différenciation desdites cellules souches humaines, et ladite perfusion étant effectuée à un taux suffisant pour obtenir la prolifération desdites cellules souches humaines dans ladite chambre de réaction, lesdits moyens comprenant un premier moyen pour introduire dans ladite chambre de réaction, et l'en éliminer, un milieu nutritif permettant l'élimination des produits métaboliques, le réapprovisionnement en substances nutritives épuisées et le maintien dudit réacteur dans des conditions physiologiquement acceptables ; lesdits moyens comprenant en outre un second moyen pour contrôler l'effluent provenant de ladite chambre de réaction.

16. Bioréacteur selon la revendication 15, dans lequel lesdites cellules du stroma adhérent à un substrat de protéines, ledit substrat de protéine étant une membrane recouverte de protéines avec des pores d'une taille comprise dans la gamme d'environ 1 à 5 µm, lesdites cellules du stroma adhérant à un côté de ladite membrane et lesdites cellules hématopoïétiques humaines étant présentes sur le côté opposé.

17. Bioréacteur selon la revendication 15, dans lequel ledit premier moyen pour introduire et éliminer ledit milieu nutritif comprend :
un réservoir de milieux pour stocker des milieux ;
un moyen pour transporter les milieux neufs dans ledit réservoir et éliminer partiellement les milieux utilisés dudit réservoir ;
un moyen pour transporter des milieux depuis ledit réservoir jusqu'au dit bioréacteur et depuis ledit bioréacteur jusqu'au dit réservoir ;
un moyen pour oxygéner lesdits milieux avant de les introduire dans ledit bioréacteur ; et
un moyen pour contrôler la composition desdits milieux dans ledit bioréacteur.
